# EUROPEAN PATENT APPLICATION

(11) **EP 2 327 434 A1**
(43) Date of publication of application: **01.06.2011**
(21) Application number: 09812954.7
(22) Date of filing: 07.07.2009
(51) Int. Cl.: A61M 5/158

(54) **MEDICAL DEVICE**

(30) Priority: 10.09.2008 JP 2008232156
(71) Applicant: Terumo Kabushiki Kaisha, Tokyo 151-0072 (JP)
(72) Inventor: TANABE, Hidenori, Nakakoma-gun Yamanashi 409-3853 (JP); KOBAYASHI, Ryoji, Nakakoma-gun Yamanashi 409-3853 (JP); MURASHITA, Takato, Nakakoma-gun Yamanashi 409-3853 (JP)
(74) Representative: TBK
(86) International application number: PCT/JP2009/062375
(87) International publication number: WO 2010/029806

(57) **Abstract**

A medical device which comprises an indwelling needle assembly comprising an inner needle which has a sharp needle tip at the end thereof and a hollow outer needle into which the inner needle is inserted and a suction means by which blood is sucked through the indwelling needle assembly in an indwelling state where the indwelling needle assembly is pierced into a living body and indwells there, wherein the suction means is provided with a suction power-controlling system which controls the suction power thereof. Between the outer peripheral face of the inner needle and the inner peripheral face of the outer needle of this medical device, a space communicating with the suction means is formed along the longitudinal direction of the indwelling needle assembly and blood flows into this space. In this space, the suction power controlled by the suction power-controlling system is set lower by 5 mmHg or more than the atmospheric pressure.

## Description

### Technical Field

The present invention relates to a medical device.

### Background Art

When an infusion is performed on a patient, or in other similar situations, an indwelling needle connected to an infusion line is made to puncture a blood vessel of the patient and the needle is left indwelling in the patient's blood vessel during the operation.

Such an indwelling needle assembly is composed of a hollow outer needle, an outer needle hub secured to a proximal end of the outer needle, an inner needle that is inserted in the outer needle and which has a sharp needle tip at a distal end thereof, and an inner needle hub secured to a proximal end of the inner needle (see, for example, Patent Document 1).

When the indwelling needle assembly is made to puncture the patient's blood vessel, the puncturing operation is performed in a condition where the inner needle is inserted in the outer needle, and the needle tip of the inner needle protrudes from the distal end of the outer needle.

Once the needle tip of the inner needle has reached the inside of the blood vessel, blood flows into the inner needle through the opening at the needle tip, passes through a lumen of the inner needle and flows into the inside of the transparent inner needle hub (flashback). Consequently, it can be confirmed (visually checked) that the inner needle has captured the blood vessel.

After flashback is confirmed, the outer needle is advanced and is inserted into the blood vessel.

Next, while gripping the outer needle by hand, the inner needle is pulled out of the outer needle. Then, an infusion line is connected to the outer needle hub, whereby an infusion agent is administered through the infusion line.

However, the indwelling needle assembly described in Patent Document 1 has the following problem. In cases where a patient is in a state of an extremely low blood pressure, for example, in a state of shock, a cardiopulmonary arrest state or the like, even if the needle tip of the inner needle has reached the inside of the patient's blood vessel and captured the blood vessel, blood may be unable to flow into the lumen of the inner needle, and, consequently, it may be impossible to confirm flashback. In addition, if the indwelling needle assembly is simply equipped with a device such as a suction pump for forcibly sucking the blood for the purpose of confirming the flashback, the indwelling needle assembly would be extremely complicate in configuration and be poor in operability.
Patent Document 1: International Publication No. WO 2006/027923 A1

### Disclosure of Invention

It is an object of the present invention to provide a medical device which is simple in configuration, in which a suction power is maintained for a predetermined period of time and by which flashback indicative of capturing of a blood vessel can be confirmed speedily.

In order to attain the above object, according to the present invention, there is provided
a medical device including:
an indwelling needle assembly which includes an inner needle having a sharp needle tip at a distal end thereof and a hollow outer needle into which the inner needle is inserted; and
suction means by which blood is sucked through the indwelling needle assembly in an indwelling state where the indwelling needle assembly is made to puncture a living body and to indwell therein;
wherein the suction means has a suction power-controlling mechanism for controlling suction power thereof.

With the structure, the suction power of the suction means is suitably controlled by the suction power-controlling mechanism, and the suction power is maintained for a predetermined period of time. As a result, even when a patient is in a state of an extremely low blood pressure, such as, for example, a state of shock or a cardiopulmonary arrest state, the patient's blood can be assuredly sucked through the indwelling needle assembly, and, therefore, flashback indicative of capturing of the blood vessel can be confirmed speedily.

In addition, according to the present invention, the suction power-controlling mechanism is arranged at a position different from the position of the indwelling needle assembly. Thereby, the configuration of the medical device is simplified, as compared to a case where a device such as a suction pump is simply disposed in the indwelling needle assembly.

Further, in the medical device of the present invention, preferably, a gap communicating with the suction means is formed between an outer peripheral surface of the inner needle and an inner peripheral surface of the outer needle, along a longitudinal direction of the indwelling needle assembly, and blood flows into the gap.

Thus, blood flows into the gap reliably, and the blood that has flowed into the gap enables flashback to be confirmed.

In addition, in the medical device of the present invention, preferably, in the gap, the suction power controlled by the suction power-controlling mechanism is 5 mmHg or more lower than the atmospheric pressure.

Thus, even when a patient is in a state of an extremely low blood pressure such as, for example, a state of shock or a cardiopulmonary arrest state, blood can be sucked assuredly through the indwelling needle assembly, and, therefore, flashback indicative of capturing of the blood vessel can be confirmed swiftly.

Further, in the medical device of the present invention, preferably, the suction power acts before the indwelling state is established.

Thus, when the indwelling needle device has captured a blood vessel, blood is instantaneously sucked from the blood vessel, and, therefore, flashback indicative of the capturing of the blood vessel can be confirmed speedily.

In addition, in the medical device of the present invention, preferably, the suction power-controlling mechanism has an orifice.

This ensures that the suction power can be controlled with a simple configuration.

Further, in the medical device of the present invention, preferably, the suction power-controlling mechanism has a plurality of the orifices having different inside diameters, and is capable of selecting one of the plurality of the orifices.

This ensures that the degree of control of the suction power can be changed according to the magnitude of the inside diameter of the selected orifice.

In addition, in the medical device of the present invention, preferably, the suction means is configured to maintain the suction power thereof for a predetermined period of time.

This ensures that the suction power is assuredly maintained even after the indwelling state has been established. Accordingly, blood can be sucked reliably, and flashback indicative of capturing of the blood vessel can be confirmed.

Further, in the medical device of the present invention, preferably, the suction means has a container in which a reduced-pressure condition is maintained.

This ensures that the suction means can be made simple in configuration, and blood can be sucked easily and reliably by the suction power of the container.

In addition, in the medical device of the present invention, preferably,
the suction means has a tube connected to the indwelling needle assembly, and
the tube has a connector detachably connected to the suction power-controlling mechanism.

This ensures that the connector detached from the suction power-controlling mechanism can be connected, for example, to a connector mounted to an end portion of an infusion line for supplying an infusion (medicinal liquid), a mouth portion (distal portion) of a syringe containing a medicinal liquid therein, or the like.

Further, in the medical device of the present invention, preferably, when the connector is detached from the suction power-controlling mechanism, the detached connector is capable of being connected to an infusion line for infusion.

This ensures that infusion can be performed in a condition where the connector and the infusion line are interconnected.

In addition, in the medical device of the present invention, preferably, the suction power-controlling mechanism is configured such that the degree of control of the suction power can be changed.

This ensures that the degree of control of the suction power can be effectively changed in a case where it is necessary to change the degree of control of the suction power, for example, depending on specifications of the indwelling needle assembly.

### Brief Description of Drawings

FIG. 1 is a plan view showing a first embodiment of a medical device according to the present invention.
FIG. 2 is a longitudinal sectional view showing an outer needle, an outer needle hub, an inner needle and a tube of an indwelling needle assembly in the medical device shown in FIG. 1.
FIG. 3 is an enlarged detail view of the vicinity of a distal end of the indwelling needle assembly shown in FIG. 2.
FIG. 4 is a sectional view taken along line A-A of FIG. 1.
FIG. 5 is a longitudinal sectional view of a suction means in the medical device shown in FIG. 1.
FIG. 6 is a partial longitudinal sectional view of a suction power-controlling mechanism in the medical device shown in FIG. 1.
FIG. 7 is a partial longitudinal sectional view of a suction power-controlling mechanism in a medical device (second embodiment) according to the present invention.
FIG. 8 is a plan view showing a third embodiment of the medical device according to the present invention.

### Best Mode for Carrying Out the Invention

Next, a medical device according to the present invention will be described in detail below, based on preferred embodiments shown in the accompanying drawings.

### <First Embodiment>

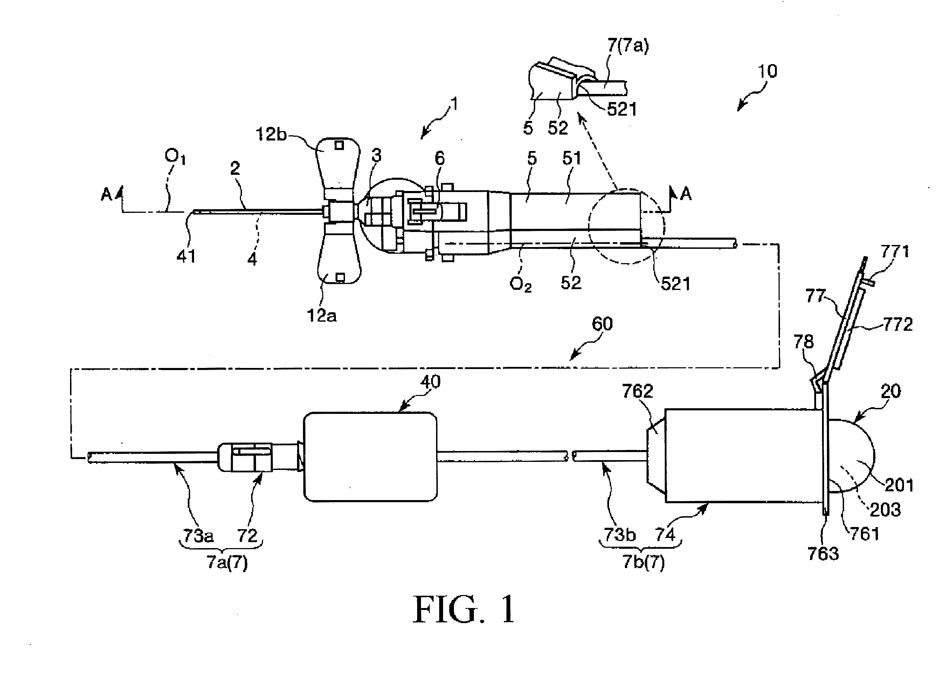

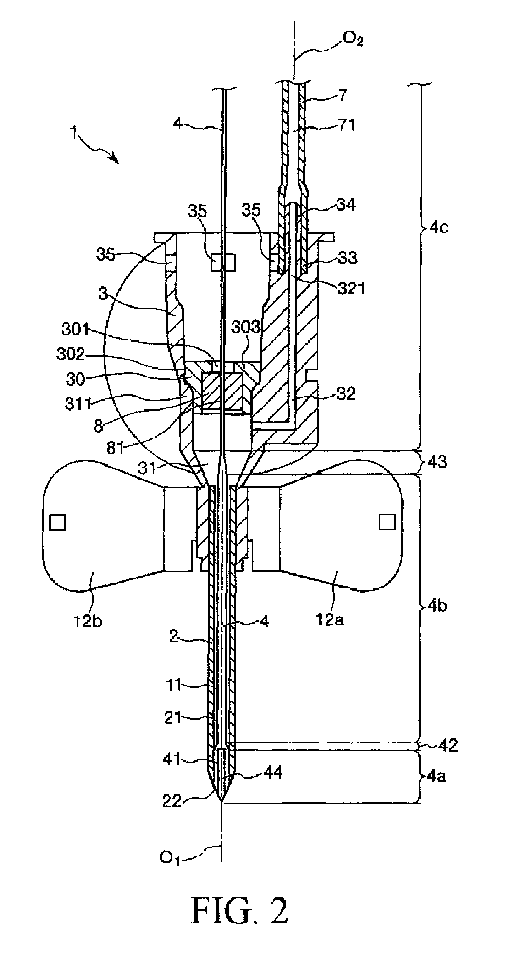

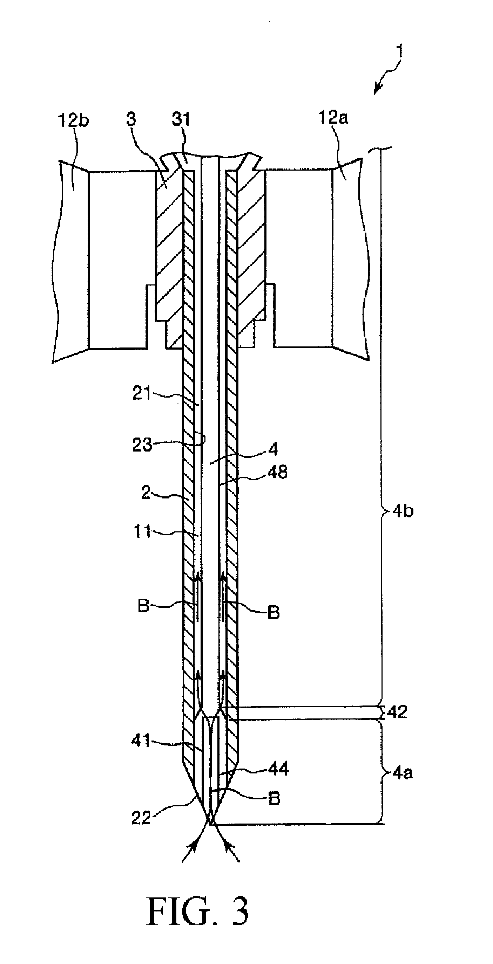

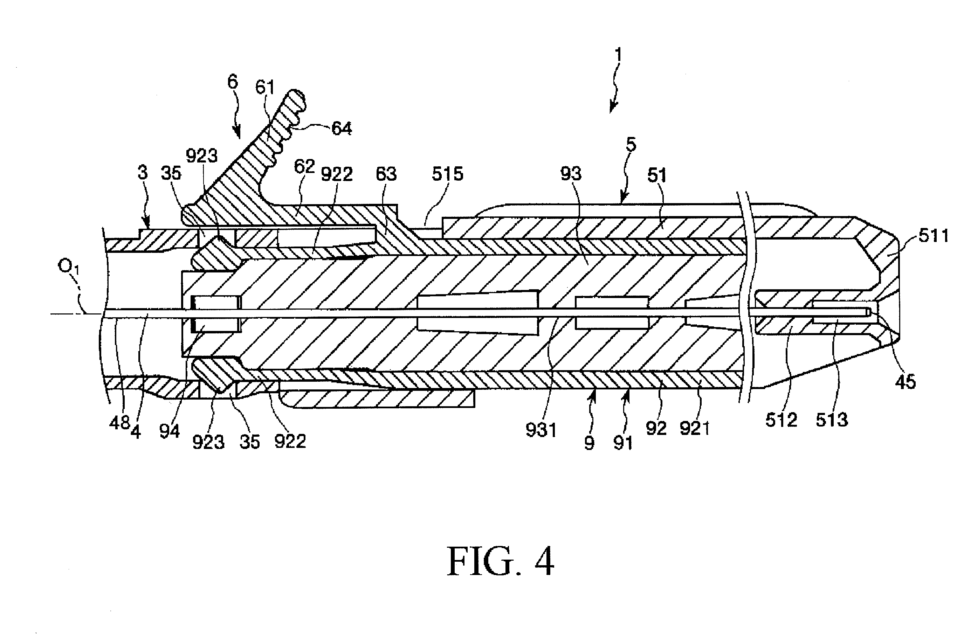

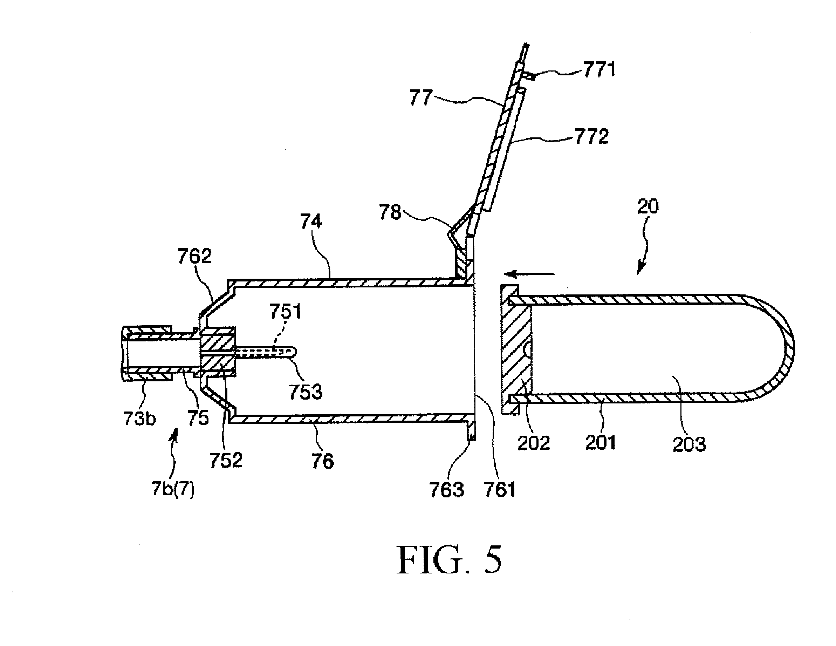

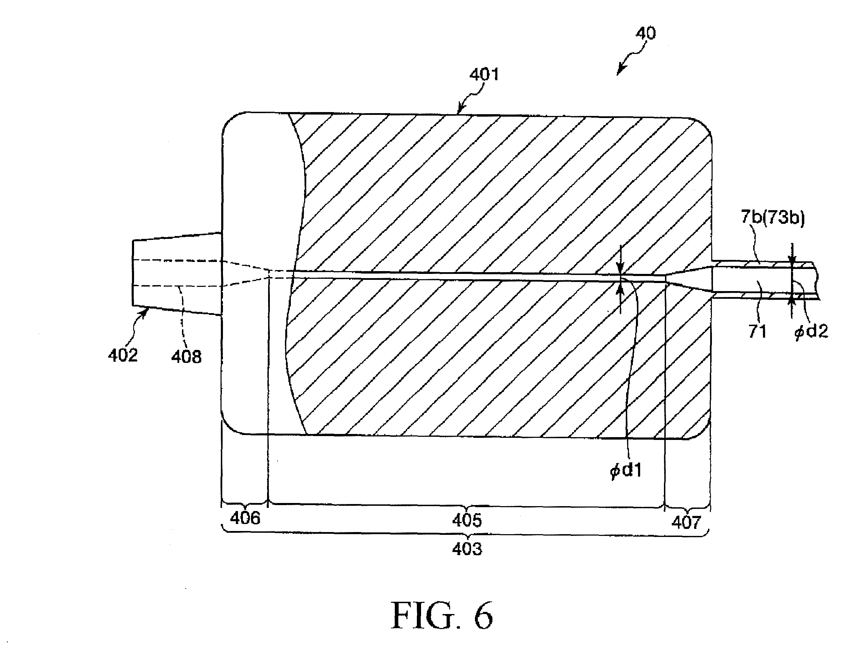

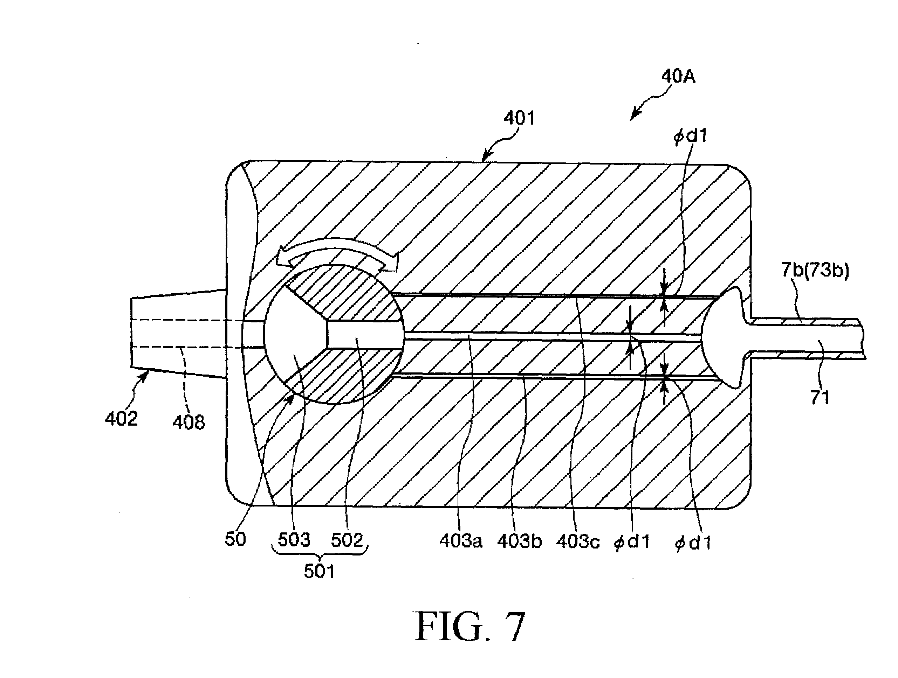

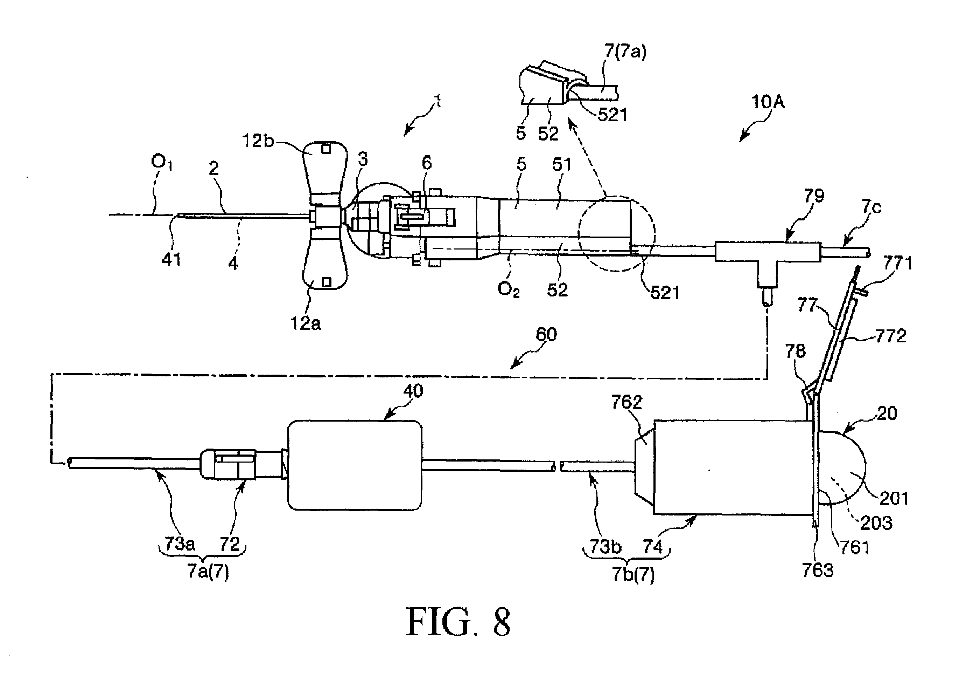

FIG. 1 is a plan view showing a first embodiment of the medical device according to the present invention. FIG. 2 is a longitudinal sectional view showing an outer needle, an outer needle hub, an inner needle, and a tube of an indwelling needle assembly in the medical device shown in FIG. 1. FIG. 3 is an enlarged detail view of the vicinity of a distal end of the indwelling needle assembly shown in FIG. 2. FIG. 4 is a sectional view taken along line A-A of FIG. 1. FIG. 5 is a longitudinal sectional view of a suction means in the medical device shown in FIG. 1. FIG. 6 is a partial longitudinal sectional view of a suction power-controlling mechanism in the medical device shown in FIG. 1. Incidentally, the right side in FIGS. 1, and 4 to 6 (also in FIGS. 7 and 8, as well) will be referred to as a "proximal" side, and the left side will be referred to as a "distal" side in the following description. Further, the upper side in FIGS. 2 and 3 will be referred to as a "proximal" side, and the lower side will be referred to as a "distal" side in the following description. In addition, in FIG. 2, an inner needle hub is omitted from illustration.

A medical device 10 shown in FIG. 1 includes an indwelling needle assembly 1 and a suction means 60. The indwelling needle assembly 1 is used in an indwelling state where it is made to puncture a living body and to indwell therein. In addition, the suction means 60 is a means for sucking blood B through the indwelling needle assembly 1 in the indwelling state. The suction means 60 includes a tube 7 having a distal portion connected to the indwelling needle assembly 1, a reduced-pressure container (a reduced-pressure tube) 20 serving as a suction source to be connected to a proximal portion of the tube 7, and a suction power-controlling mechanism 40 which is arranged at an intermediate portion of the tube 7 and which controls the suction power of the reduced-pressure container 20. Next, configurations of these parts will be described below.

The indwelling needle assembly shown in FIGS. 1 and 2 has a hollow outer needle 2, an outer needle hub 3 fixed to a proximal portion of the outer needle 2, a compression member 30 mounted to the outer needle hub 3, a seal member 8 provided on the outer needle hub 3 through the compression member 30, an inner needle 4 inserted in the outer needle 2, and an inner needle hub 5 fixed to a proximal portion 45 of the inner needle 4.

As the outer needle 2, one having a certain degree of flexibility preferably is used. The material constituting the outer needle 2 is preferably a resin material, particularly a flexible resin material. Specific examples of such materials include fluoro-resins such as PTFE, ETFE, PFA, etc., olefin resins such as polyethylene, polypropylene, etc., and mixtures thereof, polyurethane, polyesters, polyamides, polyether nylon resin, and mixtures of olefin resins with ethylene-vinyl acetate copolymer.

The outer needle 2 preferably may, wholly or partially, be formed so as to enable the inside thereof to be visible. Further, the material constituting the outer needle 2 may be admixed with a radiopaque contrast agent, such as barium sulfate, barium carbonate, bismuth carbonate, tungstic acid, etc., thereby making the outer needle opaque to radiation.

To a proximal portion of the outer needle 2, the outer needle hub 3 is firmly attached (fixed) in a liquid-tight manner by a method such as caulking, fusing (heat fusing, high-frequency wave fusing or the like), adhesion with an adhesive, etc.

The outer needle hub 3 is composed of a substantially tubular member, and the interior 31 thereof communicates with a lumen 21 of the outer needle 2.

In a portion (part) on the right side in FIG. 2 of the outer needle hub 3, there is formed a flow path 32 which has one end which opens toward the interior 31 of the outer needle hub 3. The flow path 32 is substantially L-shaped, and the other end thereof opens at a recess 33 recessedly formed in the proximal end of the outer needle hub 3, so as to form an opening 321. In addition, at a distal face (bottom face) of the recess 33, a ring-shaped projection (connecting portion) 34 is formed that projects toward the proximal side in such a manner as to surround the opening 321.

This projection 34 is inserted into a lumen 71 in a distal portion of the tube 7, whereby one end portion (distal portion) of the tube 7 is connected to the outer needle hub 3. Thus, a liquid, such as a medicinal liquid, can be supplied into the outer needle 2 (outer needle hub 3) through the tube 7.

Further, as shown in FIG. 2, on the left and right sides of the outer needle hub 3, a pair of wings 12a and 12b are formed, either by connecting separate component parts to the outer needle hub 3 or integrally with the outer needle hub 3. In this embodiment, the wings 12a and 12b are flexible and can be opened and closed by bending or curving portions of the wings 12a and 12b that are proximate to adjoining regions thereof with the outer needle hub 3. However, the present invention is not limited in this regard.

When the outer needle 2 and the inner needle 4 are made to puncture a blood vessel or the like, the wings 12a and 12b are placed in a closed state by pinching them together with fingers, so that the puncturing operation can be performed. In addition, instead of pinching the wings 12a and 12b, the inner needle hub 5 may be pinched by a thumb and a middle finger in order to perform a puncturing operation, and when the distal end of the outer needle 2 has reached the inside of the blood vessel, a finger hold part 6 (described later) may be pushed by an index finger in order to advance the outer needle hub 3, whereby only the outer needle 2 can be advanced into the blood vessel. When the outer needle 2 is set in an indwelling state with respect to a skin (living body), the wings 12a and 12b are placed in an opened state, and the wings 12a and 12b are fixed to the skin using a pressure sensitive adhesive tape or the like.

In addition, at a proximal portion of the outer needle hub 3, there are formed four holes (recesses) 35, into which projections 923 of four projecting parts 922 of a protector cover 92 of a protector 9, to be described later, are inserted. The holes (recesses) 35 are arranged at regular angular intervals about the axis of the outer needle 2.

The inner needle 4, having a sharp needle tip 41 at the distal end thereof, is inserted into the outer needle 2. As shown in FIGS. 1 and 2, the indwelling needle assembly 1 is used in a state where the inner needle 4 is inserted into the outer needle 2, and the inner needle hub 5 (described later) and the outer needle hub 3 are in abutment on each other (i.e., a state where the needle tip 41 protrudes from a distal opening 22 of the outer needle 2). Hereinafter, such a state will be referred to as an "assembled state."

The length of the inner needle 4 is set so that, in the assembled state, at least the needle tip 41 protrudes from the distal opening 22 of the outer needle 2. Further, in the assembled state, a gap 11 is formed between an outer peripheral surface 48 of the inner needle 4 and an inner peripheral surface 23 of the outer needle 2 (see FIGS. 2 and 3). As shown in FIG. 2, the gap 11 extends along the longitudinal direction of the indwelling needle assembly 1 (the inner needle 4 and the outer needle 2), and communicates with the lumen 71 of the tube 7 through the interior 31 of the outer needle hub 3 and the flow path 32.

The inner needle 4 may be either a hollow needle or a solid needle. In the case that the inner needle 4 is a solid needle, sufficient strength can be secured although the outside diameter of the needle is small. In addition, if the inner needle 4 is a solid needle, there is no danger that blood B will remain inside the inner needle 4, or that blood B might flow out therefrom, at a time of discarding the inner needle 4 after an operation has been completed. Thus, high safety is ensured.

In addition, in the case that the inner needle 4 is a hollow needle, upon puncturing a blood vessel by the inner needle 4, blood B flows into the hollow portion of the inner needle 4, whereby flashback of the blood B can be confirmed. In this connection, however, if the inner needle 4 is a solid needle, the blood B flows into the gap 11 formed between the inner needle 4 and the outer needle 2, so that flashback of the blood B can be confirmed more quickly through the outer needle 2 (see FIG. 3).

Incidentally, the inner needle 4 can have a configuration of having both a hollow part and a solid part (for example, a configuration may be provided in which part of the lumen of a hollow needle is filled, so as to make the inner needle hollow at the distal side and solid at the proximal side thereof). In this case, when the entirety of the inner needle 4 is composed of a single member, the inner needle 4 can be reduced in cost.

In addition, although the inner needle 4 may be constant in outside diameter, in the configuration shown in the drawings, the inner needle 4 has a plurality of sections (in this embodiment, three sections) that differ in outside diameter. More specifically, the inner needle 4 has a maximum outside diameter section 4a having a maximum outside diameter on a distal side (distal portion), a minimum outside diameter section 4c having a minimum outside diameter on a proximal side, and an intermediate outside diameter section 4b, the outside diameter of which is between that of the maximum outside diameter section 4a and the minimum outside diameter section 4c, and which located between the sections 4a and 4c.

Further, the inner needle 4 is formed with a first varying outside diameter section 42, which has a continuously varying outside diameter and which is located at a boundary portion between the maximum outside diameter section 4a and the intermediate outside diameter section 4b, and a second varying outside diameter section 43, which has a continuously varying outside diameter and which is located between the intermediate outside diameter section 4b and the minimum outside diameter section 4c.

At each of the varying outside diameter sections 42 and 43, the outside diameter of the inner needle 4 may vary stepwise. However, a configuration in which the outside diameter varies continuously (i.e., in a tapered manner) over such sections is advantageous for the following reason. In this manner, it is ensured that, when the inner needle 4 is pulled out from the outer needle 2, each of the varying outside diameter sections 42 and 43 can be prevented from becoming caught by a distal edge portion of a slit 81 formed in a seal member 8 (described later), the protector 9, or the like. Therefore, the operation of pulling the inner needle 4 out from the outer needle 2 can be carried out more smoothly and assuredly.

Incidentally, the varying outside diameter sections 42 and 43 may each be formed at a time when the inner needle 4 is produced. Alternatively, steps, which are formed inevitably upon formation of a later-described groove 44, may be utilized.

In addition, the maximum outside diameter section 4a has an outside diameter that is set approximately equal to the inside diameter of the outer needle 2, so that the maximum outside diameter section 4a makes close contact with the inner surface of the outer needle 2, in the condition where the inner needle 4 is inserted into the outer needle 2. On an outer peripheral portion of the maximum outside diameter section 4a (distal portion), the groove (flow path) 44 is recessed along the longitudinal direction of the inner needle 4. The groove 44 permits, in a condition where the inner needle 4 is inserted into the outer needle 2, the distal opening 22 of the outer needle 2 to communicate with the interior 31 of the outer needle hub 3. The groove 44 also functions as a flow path for blood B (body fluid) when a blood vessel is punctured, for example. This enables flashback of blood B to reliably be confirmed.

The material constituting the aforementioned inner needle 4 may be a metallic material such as, for example, stainless steel, aluminum or aluminum alloys, titanium or titanium alloys, etc.

To the proximal portion 45 of the inner needle 4, the inner needle hub 5 is firmly attached (fixed). As shown in FIG. 1, the inner needle hub 5 has a tubular protector-containing section (connection member containing section) 51 in which the inner needle 4 is inserted and in which the protector 9 is contained (disposed) in the assembled state. The inner needle hub 5 also includes a tube-containing section 52, which is provided at a lateral side (lower side in FIG. 1) of the protector-containing section 51, and in which a distal side of the tube 7 is contained (disposed) in the assembled state.

As shown in FIG. 4, a proximal end wall 511 is formed at the proximal end of the protector-containing section 51. The proximal end wall 511 is formed with a projection part (fixation part) 512 projecting in the distal direction. The projection part 512 is formed therein with a hole 513 into which the proximal portion 45 of the inner needle 4 is inserted. The proximal portion 45 of the inner needle 4 is inserted into the hole 513, and is firmly attached (fixed) to the projection part 512.

Further, in the assembled state, the protector 9 is located in the protector-containing section 51, and it can be moved relative to the protector-containing section 51.

In addition, in the assembled state, the tube 7 is inserted into the tube-containing section 52 of the inner needle hub 5, whereby the tube 7 can be prevented from obstructing operations of the indwelling needle assembly 1 (see FIG. 1).

The tube-containing section 52 is formed with a groove 521 therein, and the tube 7 is disposed inside the groove 521. The portion (part) defining (constituting) the groove 521 functions as a guide means by which the tube 7 is guided. The guide means, or the portion defining the groove 521, guides the tube 7 in such a manner that a center axis (axis) O₂ at a distal portion of the tube 7 is substantially parallel to the longitudinal direction of the inner needle hub 5 (the center axis O₁ of the outer needle 2).

Thus, the tube 7 is connected to a proximal portion of the outer needle hub 3. Also, the center axis O₁ of the outer needle 2 and the center axis O₂ at the distal portion of the tube 7 are substantially parallel to each other in the assembled state. In other words, the tube 7 protrudes in the proximal direction from the proximal end of the outer needle hub 3.

Further, when the tube 7 is detached from the inner needle hub 5 upon pulling the inner needle 4 out from the outer needle 2, the tube 7 can be detached easily and speedily by means of the groove 521 (through the groove 521).

Examples of methods for fixing the inner needle 4 to the inner needle hub 5 include fitting, caulking, fusing, adhesion with an adhesive, etc., as well as combinations of these methods. In addition, in the case that the inner needle 4 is hollow, sealing is required to prevent backward flowing blood B, for example, upon puncturing of a blood vessel, from flying out from the proximal end of the inner needle 4.

The inner needle hub 5 and the aforementioned outer needle hub 3 each preferably are formed from a transparent (colorless transparent), colored transparent or semitransparent resin so as to enable the insides thereof to be visible. This ensures that when the outer needle 2 has captured (i.e., has securely reached the inside of) a blood vessel, flashback of the blood B that flows in through the groove 44 of the inner needle 4, as described above, can be confirmed by visual observation. In addition, if the inner needle 4 is solid, the entire portion of the blood B, in which undergoes flashback under pressure inside the blood vessel, for example, flows back through the groove 44, so that better visibility and confirmation of flashback can be ensured.

The materials constituting the outer needle hub 3, the inner needle hub 5 and the wings 12a, 12b are not particularly limited. Examples of suitable materials include various resin materials including polyolefins such as polyethylene, polypropylene, ethylene-vinyl acetate copolymer, etc., polyurethane, polyamides, polyesters, polycarbonate, polybutadiene, polyvinyl chloride, and polyacetal.

Further, in the indwelling needle assembly 1, the cylindrical (block-formed) seal member 8 is disposed in the interior 31 of the outer needle hub 3, with the compression member 30 interposed therebetween. The seal member 8 is formed with a slit 81 into which the inner needle 4 can be inserted and which is closed by itself when the thus inserted inner needle 4 is pulled off. In other words, the slit 81 has a sealing function. The slit 81 is formed in a substantially central portion of the seal member 8 so as to penetrate the seal member 8 along the longitudinal direction of the seal member 8.

The slit 81 is in the shape of a straight line segment. This enables the slit 81 to easily be brought from a closed state into an open state. Consequently, the inner needle 4 can be inserted smoothly into and passed through the seal member 8 (slit 81). More specifically, as will be described later, when the outer needle 2 is advanced using the inner needle 4 as a guide, frictional resistance between an outer surface of the inner needle 4 (the minimum outside diameter section 4c) and an inner surface of the slit 81 can be reduced. Accordingly, operability of the indwelling needle assembly 1 is enhanced when a puncturing operation is performed.

The seal member 8 also has a self-closing property, such that the slit 81 becomes closed by an elastic force (restoring force) of the seal member 8 itself when the inner needle 4, which is inserted in the slit 81 in the assembled state, is pulled out from the slit 81. This enables leakage of liquid from the proximal end of the outer needle hub 3 upon pulling out of the inner needle 4 to be prevented, and further maintains an aseptic condition inside the outer needle hub 3.

In addition, as shown in FIG. 2, in the assembled state, the minimum outside diameter section 4c of the inner needle 4 is located inside the slit 81,. This ensures a small contact area between the outer surface of the minimum outside diameter section 4c and the inner surface of the slit 81, whereby frictional resistance between such surfaces can be kept small. Further, it is possible to prevent the seal member 8 (slit 81) from acquiring a semi-permanent deformation, which could lower the sealing performance thereof.

Examples of suitable materials constituting the seal member 8 include various elastic materials such as various rubber materials (particularly, vulcanized rubbers) such as natural rubber, isoprene rubber, butyl rubber, butadiene rubber, styrene-butadiene rubber, urethane rubber, nitrile rubber, acrylic rubber, fluoro-rubber, silicone rubber, etc., various thermoplastic elastomers based on urethane, polyester, polyamide, olefin, styrene or the like, as well as mixtures of the same.

The compression member 30 is a member for containing the seal member 8 therein and compressing an outer peripheral portion of the seal member 8. As shown in FIG. 2 (and in FIGS. 4 to 9, as well), the compression member 30 has a bottomed tubular shape (a tubular body). The compression member 30 has a hole 301 provided in a bottom portion 303 thereof, and a flange (enlarged diameter portion) 302 provided at the outer periphery of the bottom portion 303.

The hole 301 is provided substantially in the center of the bottom portion 303. The hole 301 has an inside diameter which is set slightly larger than the outside diameter of the maximum outside diameter section 4a of the inner needle 4 so that the inner needle 4 (ranging from the minimum outside diameter section 4c to the maximum outside diameter section 4a) can be passed therethrough.

The flange 302 is a part obtained by enlarging the outside diameter of the bottom portion 303.

In addition, the inside diameter of the compression member 30 is set smaller than the outside diameter of the seal member 8. Thereby, when the seal member 8 is pressed (fitted) into the compression member 30 (hereinafter this state will be referred to as a "press-fitted state"), the seal member 8 is compressed in a radial direction thereof. In the press-fitted state, the compression member 30 and the seal member 8 are disposed in the interior 31 of the outer needle hub 3.

As shown in FIG. 2, the outer needle hub 3 is provided in the interior 31 with a stepped part 311 where the inside diameter thereof is varied. On the stepped part 311, a distal surface of the flange 302 of the compression member 30 abuts. In addition, the compression member 30 is fitted into the interior 31 of the outer needle hub 3. By thus disposing the compression member 30, positioning of the compression member 30 relative to the outer needle hub 3 is achieved.

By the thus-constructed compression member 30, in the seal member 8 placed in the assembled state, the inner surfaces of the slit 81 are securely held in close contact with the outer surface of the inner needle 4, and also the inner surfaces of the slit 81 are securely held in close contact with each other. Thus, the sealing function of the slit 81 (the seal member 8) is reliably maintained. That is, the sealing function is reliably prevented from lowering. Consequently, leakage of a liquid such as blood B or a medicinal liquid through the slit 81 can be reliably prevented. In addition, an aseptic condition inside the outer needle hub 3 (i.e., an aseptic condition of the interior 31) can be maintained.

Also, in the condition where the inner needle 4 is pulled out from the slit 81, the seal member 8 has the inner surfaces of the slit 81 that are reliably held in close contact with each other. This ensures that the sealing function of the slit 81 is maintained reliably, so that leakage of liquid through the slit 81 can be securely prevented. In addition, like in the assembled state as above-mentioned, an aseptic condition inside the outer needle hub 3 can be maintained.

Further, since the seal member 8 is cylindrical in shape as above-mentioned, the compression member 30 can compress the seal member 8 evenly along the circumferential direction thereof. Thus, non-uniform contact between the inner surfaces of the slit 81 and the outer surface of the inner needle 4 can be prevented from occurring in the assembled state. Therefore, the sealing function of the seal member 8 is maintained reliably.

In addition, the inside diameter of the compression member 30 (tubular body) is preferably smaller than the outside diameter of the seal member 8 by 1 to 30%, more preferably by 10 to 15%. In other words, a ratio of compression of the seal member 8 by the compression member 30 is preferably 1 to 30%, more preferably 10 to 15%.

If the inside diameter of the compression member 30 is smaller than the above-mentioned lower limit, the seal member 8 is not sufficiently compressed, so that the sealing function may become insufficient. In contrast, if the inside diameter of the compression member 30 is larger than the above-mentioned upper limit, the seal member 8 is compressed excessively, so that it becomes difficult to insert the inner needle 4 into the slit 81, or it becomes difficult to pull the inserted inner needle 4 out from the slit 81.

Examples of suitable materials constituting the compression member 30 include various resin materials such as polyolefins such as polyethylene, polypropylene, ethylene-vinyl acetate copolymer, etc., polyurethane, polyamides, polyesters, polycarbonate, acrylic resin, polybutadiene, polyvinyl chloride, etc., and metallic materials such as stainless steel, aluminum, aluminum alloys, titanium, titanium alloys, etc.

Further, the indwelling needle assembly 1 includes the protector 9, by which at least the needle tip 41 of the inner needle 4 is covered when the inner needle 4 has been pulled out from the outer needle 2. The protector 9 is contained in the protector-containing section 51 of the inner needle hub 5 in such a manner that it can be moved along the longitudinal direction of the inner needle 4. The protector 9 will be described below.

The protector 9 is detachably connected to the outer needle hub 3. As shown in FIG. 4, the protector 9 includes a protector body 91, and a shutter member (shutter means) 94 provided inside the protector body 91.

The protector body 91 includes a protector cover 92, and an inner member 93 that is inserted in the protector cover 92. The protector cover 92 and the inner member 93 are configured so as to be movable relative to each other. Further, the protector cover 92 and the inner member 93 can assume a state where movement thereof is inhibited, as well as a state where movement thereof is permitted.

The protector cover 92 includes a cover body part 921, which is pipe-like (tubular) in shape, and four projecting parts 922 formed at a distal portion of the cover body part 921, which project in the distal direction. A distal side of each of the projecting parts 922 is inserted into a proximal portion of the outer needle hub 3. At a distal portion of each of the projecting parts 922, there is formed a projection 923, which is inserted into the hole 35 formed in a proximal portion of the outer needle hub 3, and which becomes caught on an edge portion confronting the hole 35.

As shown in FIG. 4, when the inner member 93 is inserted into the protector cover 92 and a distal portion of the inner member 93 is located at the part (position) of the projections 923 of the projecting parts 922 of the protector cover 92, the projections 923 are inhibited by the inner member 93 from being moved (displaced) along the direction of the center axis (axis) of the inner needle 4, so that engagement of the projections 923 with the edge portions confronting the holes 35 (i.e., a condition in which the projections 923 are caught on the edge portions confronting the holes 35) is held (maintained). Consequently, the connection state between the protector 9 and the outer needle hub 3 is held.

Starting from this condition, when the inner needle hub 5 is moved in the proximal direction, the inner needle 4 is moved accordingly. In this instance, the protector 9 is still held in the connected state, so that the protector 9 does not move in the proximal direction. When the inner needle hub 5 is further moved in the proximal direction, the maximum outside diameter section 4a of the inner needle 4 engages with the inner member 93 of the protector 9, so that the inner member 93 can be moved in the proximal direction relative to the protector cover 92. When a distal portion of the thus-moved inner member 93 reaches the proximal side of the projections 923 of the protector cover 92, the projections 923 are capable of moving toward the center axis of the inner needle 4. In this condition, when the protector cover 92 is moved in the proximal direction relative to the outer needle hub 3, the projecting parts 922 are deformed (deflected) in the direction approaching the center axis of the inner needle 4, whereby engagement of the projections 923 with the edge portions confronting the holes 35 is released, and the protector 9 is disengaged from the outer needle hub 3. In this instance, by a structure for engagement or the like, for example, the inner member 93 is prevented from being separated away (disengaged) from the protector cover 92.

The material constituting the protector cover 92 is not particularly limited. For example, materials identical or similar to those mentioned above as materials for the outer needle hub 3 and the inner needle hub 5 can be used.

The inner member 93 is inserted into the above-mentioned protector cover 92. The inner member 93 is pipe-like (tubular) in shape. More specifically, the inner member 93 is formed at a central portion thereof with an inner needle passage 931, into which the inner needle 4 is inserted. The inner needle passage 931 penetrates the inner member 93 from a proximal end toward a distal end of the inner member 93. The shutter member 94 is contained in an intermediate portion of the inner needle passage 931, which is located at a distal portion of the inner member 93. The shutter member 94 is such a member that when the inner needle 4 is moved in the proximal direction and the maximum outside diameter section 4a of the inner needle 4 engages with the inner member 93 of the protector 9 as above-mentioned, the needle tip 41 is located on the proximal side relative to the shutter member 94, whereby the needle tip 41 is inhibited from passing through the inner needle passage 931 in the distal direction.

The material constituting the inner member 93 is not specifically limited. For example, materials identical or similar to those mentioned above as materials for the outer needle hub 3 and the inner needle hub 5 can be used.

According to the protector 9, as described above, after use thereof, the needle tip 41 of the inner needle 4 can be covered speedily and safely through a simple operation. In addition, by the action of the shutter member 94, the needle tip 41, once covered, is prevented from protruding from the distal end of the protector body 91 (inner member 93) of the protector 9. Consequently, upon disposal of the inner needle 4 or the like, or in other similar situations, an accident in which a worker or the like pierces his or her finger or the like with the needle tip 41 by mistake, is prevented from occurring, and high safety is secured.

In addition, the indwelling needle assembly 1 may be provided with a disengagement prevention means for preventing the needle tip 41 of the inner needle 4 from being disengaged from the protector 9 when the needle tip 41 is covered with the protector 9.

Further, as shown in FIG. 4 (and in FIG. 1, as well), the protector cover 92 of the protector 9 is formed (provided) with a projecting finger hold part (tab) 6, which is pushed by a finger for moving the outer needle 2 in the distal direction relative to the inner needle 4. The protector cover 92 and the finger hold part 6 are formed integrally (or in connection). In addition, the finger hold part 6 projects in an upward direction through a cutout 515 formed in a distal portion of the protector-containing section 51 of the inner needle hub 5. The term "upward direction" means a direction in which a skin (body surface) in the vicinity of a part of a patient (a person for whom the indwelling needle assembly 1 is used) to be punctured by the outer needle 2 (the inner needle 4) is directed, namely, a direction from the skin side toward the side of the indwelling needle assembly 1, when the indwelling needle assembly is used. In other words, the upward direction means a direction in which a cutting edge (not shown) formed at the needle tip 41 of the inner needle 4 is directed.

The finger hold part 6 is provided on the proximal side thereof with a finger hold surface 64 on which to put a finger. The finger hold part 6 is shaped such that, when the outer needle 2 is moved in the distal direction relative to the inner needle 4, force in the upward direction (upward vertical direction) relative to the center axis (axis) O₁ of the outer needle 2, namely, force in the direction in which the finger hold part 6 projects (projection direction) (the upward direction in FIG. 4), can act on the finger hold part 6.

Thus, when the outer needle 2 is moved in the distal direction relative to the inner needle 4 in a puncturing operation, the finger hold part 6 can be pushed by a finger in the distal direction while (in the manner of lifting) the finger hold part 6 is lifted in the projection direction (in the upward direction in FIG. 4). As a result, when the outer needle 2 is moved in the distal direction, the center axis O₁ of the outer needle 2 can be prevented from being inclined relative to the center axis O₁ of the outer needle 2 in a state before the finger hold part 6 is pushed. Thus, the outer needle 2 can be moved straight along the center axis O₁ without bending, namely, can be moved in the direction of the center axis O₁. Accordingly, the outer needle 2 can be moved (advanced) smoothly, and excellent operability is obtained.

In the present embodiment, the finger hold part 6 is formed at a distal portion of the cover body part 921 of the protector cover 92. As shown in FIG. 4, the finger hold part 6 has a shape obtained by bending a plate body. More specifically, the finger hold part 6 is composed of an inclined part (inclined plate) 61, which is arranged on the distal side relative to the cover body part 921, and which is inclined toward the proximal side, a base part 63 fixed to a distal portion of the cover body part 921, and a connecting part (connecting plate) 62 that interconnects the inclined part 61 and the base part 63. A proximal-side surface of the inclined part 61 constitutes a finger hold surface 64. With the finger hold part 6, a finger is inserted between the finger hold surface 64 (the inclined part 61) and the connecting part 62, the finger is hooked on the finger hold surface 64, and the finger hold part 6 can be pushed in the distal direction while lifting up the finger hold part 6 in the projection direction thereof by the finger.

In addition, the positional relationship between the finger hold part 6 and the center axis O₁ of the outer needle 2 is not particularly limited. As shown in FIG. 1, it is preferable, however, that the finger hold part 6 is disposed on the center axis O₁ of the outer needle 2 in plan view (as shown in FIG. 1). This enables the outer needle 2 to be moved in the direction of the center axis O₁ more smoothly and reliably.

Further, the finger hold surface 64 of the finger hold part 6 is formed with a certain degree of roughness (for example, a plurality of ribs arranged side by side along an up-down direction of the finger hold surface 64) as a finger anti-slip means. This prevents the finger from slipping off during movement of the outer needle 2 in the distal direction, by pushing the finger hold part 6 with the finger.

As shown in FIG. 2 (and in FIG. 1, as well), the tube 7 is connected to a proximal portion (or a side portion) of the outer needle hub 3. The lumen 71 of the tube 7 communicates with the lumen 21 of the outer needle 2 through the flow path 32 and the interior 31 of the outer needle hub 3.

As shown in FIG. 1, the tube 7 can be divided into a first tube 7a located on the distal side and a second tube 7b located on the proximal side, with the suction power-controlling mechanism 40 interposed therebetween.

The first tube 7a has a long tube body 73a and a connector 72 attached to a proximal portion of the tube body 73a.

The tube body 73a has a distal portion connected to a proximal portion of the outer needle hub 3. The tube body 73a is formed of a flexible material, which is not particularly limited. Examples of the materials include polyolefins such as polyethylene, polypropylene, ethylene-vinyl acetate copolymer, etc., polyvinyl chloride, polybutadiene, polyamides, and polyesters, among which particularly preferred is polybutadiene. In a case in which polybutadiene is used as the material constituting the tube body 73a, appropriate flexibility, chemical resistance, and an excellent chemical adsorption preventive property can be obtained.

The connector 72 is composed of a tubular member, and a distal portion thereof is fixed to a proximal portion of the tube body 73a by, for example, adhesion (adhesion with an adhesive or by use of a solvent). In addition, the connector 72 is formed at an inner peripheral portion thereof with a female luer taper (not shown). The female luer taper has an inside diameter that gradually decreases along the distal direction. This enables the connector 72 to be fitted onto a male luer taper mouth part 402 of the suction power-controlling mechanism 40 which will be described later. Therefore, the connector 72 can be detachably connected to the mouth part 402. Alternatively, a closed connector (needleless connector) or the like may be used as the connector 72, and the closed connector or the like may be connected to the mouth part 402 of the suction power-controlling mechanism 40. When the connector 72 is detached from the mouth part 402 of the suction power-controlling mechanism 40, the detached connector 72 can be connected to a connector that is attached to an end portion of an infusion line for supplying an infusion (medicinal liquid) to be administered to a patient, a mouth part (distal portion) of a syringe containing a medicinal liquid therein, or the like. Incidentally, the material constituting the connector 72 is not particularly limited, and for example, such materials as mentioned in the above description of the outer needle hub 3 and the inner needle hub 5 can be used.

The second tube 7b has a long tube body 73b and a connector 74 attached to a proximal end of the tube body 73b.

The tube body 73b has a distal portion integrally connected to a proximal portion of a body part 401 of the suction power-controlling mechanism 40. The material constituting the tube body 73b is not particularly limited. For example, such materials as mentioned in the above description of the tube body 73a can be used.

As shown in FIG. 5, the connector 74 is a member to which the reduced-pressure container 20 is detachably connected. The connector 74 has a needle assembly 75 and a holder 76. Incidentally, in the present embodiment, the connector 74 further has a lid 77, but the present invention is not limited in this regard.

The needle assembly 75 is composed of a hollow needle (needle pipe) 751 having a sharp needle tip, a hub 752 firmly attached to a distal portion of the hollow needle 751, and a rubber sheath 753 enveloping the hollow needle 751.

The hub 752 is connected to a proximal portion of the tube body 73b. The hollow needle 751 and the tube body 73b communicate with each other through the hub 752. The material constituting the hub 752 is not particularly limited. Examples of the material include polycarbonates, polyolefins such as polyethylene, polypropylene, ethylene-vinyl acetate copolymer, etc., and modified polyolefins.

The hollow needle 751 is supported on a proximal portion of the hub 752. The hollow needle 751 is formed, for example, from a metallic material such as stainless steel, etc., and can pierce a rubber stopper 202 of the reduced-pressure container 20 when the reduced-pressure container 20 is connected to the connector 74. Thus, the reduced-pressure container 20 and the tube 7 communicate with each other through the needle assembly 75.

In addition, the rubber sheath 753 covers the hollow needle 751 in a condition where the reduced-pressure container 20 is not connected to the connector 74. This ensures, for example, that an aseptic condition of the hollow needle 751 can be maintained.

The holder 76 is a member into which the reduced-pressure container 20 is inserted and which supports the reduced-pressure container 20 therein. The holder 76 has a tubular shape with an opening part 761 at a proximal end thereof, and the reduced-pressure container 20 can be inserted through the opening part 761. The holder 76 is disposed on the outer periphery side of the hollow needle 751, and has a proximal portion 762 that is joined to the hub 752 concentrically with the hollow needle 751. In addition, a projecting flange part 763 is formed at an outer peripheral portion (edge portion) of the opening part 761. The flange part 763 is plate-like in shape.

On the flange part 763, a plate-like lid 77 is turnably supported through a hinge (turning mechanism) 78. The lid 77 can be turned relative to the holder 76 by operation of the hinge 78. Thus, the lid 77 can be placed in a first state of covering the opening part 761 and a second state (a state shown in FIGS. 1 and 5) of opening the opening part 761. When the reduced-pressure container 20 is not connected to the holder 76, the lid 77 is placed in the first state. In this state, for example, a finger is inhibited from entering the holder 76. This makes it possible, for example, to prevent a finger from entering the holder 76 accidentally. Further, when the reduced-pressure container 20 is connected to the holder 76, the lid 77 is brought into the second state.

In addition, the lid 77 has a claw 771 to be engaged with the flange part 763. With the claw 771 engaged with the flange part 763, the first state can be maintained. More specifically, the lid 77 can be prevented from being displaced into the second state in an unwilling manner.

Further, the lid 77 has a rib 772 which projects on the back surface of the lid 77 in a ring-like shape (C-shape). The rib 772 has an outside diameter approximately equal to or slightly smaller than the inside diameter of the opening part 761 of the holder 76. The rib 772 is fitted into the opening part 761 in the first state.

Incidentally, the materials constituting the holder 76 and the lid 77 are not particularly limited. For example, such materials as mentioned in the above description of the hub 752 can be used.

In addition, the configuration of the turning mechanism is not particularly limited. Examples of suitable configurations include a configuration which is composed of a thin wall part for interconnecting the flange part 763 and the lid 77, etc., in addition to the above configuration of the hinge 78.

The reduced-pressure container 20 connected to the connector 74 functions as a suction source for sucking blood B through the indwelling needle assembly 1 in an indwelling state where the indwelling needle assembly 1 is set indwelling in a patient's skin. In the present embodiment, the reduced-pressure container 20 is composed of a bottomed tubular container body 201 and the rubber stopper 202 fitted into a mouth part of the container body 201, but the present invention is not limited to this configuration. The reduced-pressure container 20 is placed in a state where the opening part of the container body 201 is sealed in a gas-tight manner with the rubber stopper 202. Thus, an internal space 203 of the reduced-pressure container 20 is hermetically sealed, and a vacuum or reduced-pressure condition is maintained. Incidentally, the reduced-pressure container 20 may have a configuration in which the opening part of the container body 201 is sealed with a film instead of the rubber stopper 202.

When the reduced-pressure container 20 is connected to the connector 74, the rubber stopper 202 is pierced by the hollow needle 751 of the connector 74. Thereby, the inside (internal space 203) of the reduced-pressure container 20 communicates with the gap 11 in the indwelling needle assembly 1 through the tube 7. In the indwelling state, blood B is sucked into the gap 11 by the action of the reduced-pressure container 20, that is, by the suction power of the reduced-pressure container 20 (see FIG. 3).

Such a reduced-pressure container 20 can be substituted, for example, by a vacuum blood collection tube (reduced-pressure blood collection tube). Such a vacuum blood collection tube preferably has a capacity (internal volume) of 0.5 to 20 cc, more preferably 5 to 13 cc. In a case where the capacity of the vacuum blood collection tube is in the above numerical value range, the pressure (internal pressure) inside the internal space 203 of the vacuum blood collection tube (reduced-pressure container 20) is generally 40 to 700 mmHg. Incidentally, in the present embodiment, if the vacuum blood collection tube is used, blood-drawing can be conducted therewith.

Meanwhile, in a case in which the suction power-controlling mechanism 40 is omitted from the medical device 10, if the above-mentioned vacuum blood collection tube is used as the reduced-pressure container 20, the magnitude of the suction power in the gap 11 in the indwelling needle assembly 1 may become excessive. This may lead to abrupt suction of blood B of a patient, and to a short suction power retention time.

In view of this, in the medical device 10, the suction power-controlling mechanism 40 for controlling (regulating) the suction power of the reduced-pressure container 20 is disposed between the indwelling needle assembly 1 and the reduced-pressure container 20, whereby the suction power in the gap 11 under the action of the reduced-pressure container 20 is controlled. This ensures that the suction power is set to an appropriate magnitude, and abrupt suction of blood B of a patient and similar problems can be prevented from occurring. The configuration and operation of the suction power-controlling mechanism 40 will be described later.

In addition, the reduced-pressure container 20 is preliminarily set in a reduced-pressure state before connected to the connector 74 of the tube 7 as above-mentioned. Accordingly, when the reduced-pressure container 20 is connected to the connector 74, a suction power is generated over a region from the reduced-pressure container 20 to the interior of the gap 11, and the suction power is maintained for a predetermined period of time. The period of time for which the suction power is maintained (suction power retention time) is not particularly limited, and for example, the suction power retention time is preferably not less than one minute, more preferably not less than three minutes.

For example, in a case where the reduced-pressure container 20 is connected to the connector 74 of the tube 7 before the indwelling needle assembly 1 is made to puncture a patient's skin, namely, before the indwelling needle assembly 1 is brought into the indwelling state, the period of time from the connection of the reduced-pressure container 20 to the connector 74 of the tube 7 until establishing of the indwelling state is in the range of several tens of seconds to two minutes, and, therefore, the suction power retention time is preferably set equal to or more than the above period of time. This ensures that the suction power is reliably maintained even after the indwelling state is established, and accordingly, blood B can be sucked assuredly, and flashback indicative of the capture of the blood vessel can be confirmed.

As shown in FIG. 1, the suction power-controlling mechanism 40 is disposed between the first tube 7a and the second tube 7b. As shown in FIG. 6, the suction power-controlling mechanism 40 has a body part 401 and a projecting mouth part 402 formed integrally with a distal portion of the body part 401.

The body part 401 is a flat-shaped member. The body part 401 is formed with an orifice 403 composed of an extremely thin flow path extending through the body part 401 in a plane direction thereof. The orifice 403 has a proximal portion communicating with the tube body 73b. In addition, the orifice 403 has a constant inside diameter section 405 located at an intermediate portion thereof, and varying inside diameter sections 406 and 407 located respectively at both end portions thereof. The constant inside diameter section 405 has an inside diameter φd1 which is constant along the longitudinal direction of the orifice 403. Further, the inside diameter φd1 of the constant inside diameter section 405 is smaller than the inside diameter φd2 of the tube bodies 73a, 73b.

The mouth part 402 is tubular in shape, and has a lumen part 408 that communicates with the orifice 403. An outer peripheral portion of the mouth part 402 is in the form of a male luer taper, which has an outside diameter gradually decreasing along the distal direction. The male luer taper (mouth part 402) is fitted into the above-mentioned female luer taper of the connector 72, whereby the suction power-controlling mechanism 40 and the first tube 7a are connected to (communicate with) each other.

The constant inside diameter section 405, which takes up most part of the orifice 403, has a length which is set as follows.

For example, in a case where the indwelling needle assembly 1 is of 24 G (gauge), a vacuum blood collection tube with an internal volume of 10 cc is used as the reduced-pressure container 20, and the inside diameter φd1 is 70 µm, the length of the constant inside diameter section 405 may be set to a value of 3 to 96 mm, preferably 12 to 24 mm. Incidentally, the indwelling needle assembly 1 of 24 G (gauge) means that it has an inside diameter of the outer needle 2 of 0.53 mm and an outside diameter (average) of the inner needle 4 of 0.45 mm.

In a case where the inside diameter φd1 and the length of the constant inside diameter section 405 are set in this manner, the suction power of the reduced-pressure container 20 is regulated by the constant inside diameter section 405 (the suction power-controlling mechanism 40). More specifically, the suction power of the reduced-pressure container 20 suffers a pressure loss in the constant inside diameter section 405, and as a result, a state where the suction power in the gap 11 in the indwelling needle assembly 1 is 5 mmHg or more lower than the atmospheric pressure can be maintained for a predetermined period of time. When the medical device 10 in this state is used, even if a patient is in a state of an extremely low blood pressure such as a state of shock or a cardiopulmonary arrest state, blood B can be reliably sucked from the patient via the indwelling needle assembly 1, and, therefore, flashback indicative of capturing of the blood vessel can be confirmed speedily.

In addition, the medical device 10 includes the indwelling needle assembly 1, the suction power-controlling mechanism 40 and the reduced-pressure container 20, which are sequentially arranged along the longitudinal direction of the tube 7. Thus, the configuration is simple, as compared to a case where a device such as a suction pump is simply disposed in the indwelling needle assembly 1.

Incidentally, the material constituting the suction power-controlling mechanism 40 is not particularly limited. For example, such materials as mentioned in the above description of the outer needle hub 3 and the inner needle hub 5 can be used.

Next, an example of a method of using the medical device 10 will be described in detail below.
[1] The medical device 10 is prepared. In the medical device 10, the indwelling needle assembly 1 is in the assembled state, and the reduced-pressure container 20 is not yet connected to the connector 74 of the tube 7.
[2] Next, the reduced-pressure container 20 is connected to the connector 74 of the tube 7 (the state shown in FIG. 1). Consequently, suction power is applied to the medical device 10 before the indwelling state is established.

Then, the wings 12a and 12b are pinched with fingers and closed, and, with the wings 12a and 12b used as a gripping part (operating part), the integrated combination of the outer needle 2 and the inner needle 4 of the indwelling needle assembly 1 is made to puncture a patient's blood vessel (vein or artery). In this case, the suction power is already applied to the gap 11 in the indwelling needle assembly 1, so that blood B is sucked from the blood vessel instantaneously when the blood vessel is captured by the indwelling needle assembly 1. As a result, flashback indicative of the capture of the blood vessel can be speedily confirmed in at least one site among the outer needle 2, the outer needle hub 3, the inner needle hub 5 and the tube 7 which have inside visibility. In addition, since the puncturing operation on the blood vessel is carried out by gripping the wings 12a and 12b, the puncture angle is made smaller, in other words, the outer needle 2 and the inner needle 4 are arranged more closely in parallel in relation to the blood vessel, compared to a case in which the puncturing operation is carried out by directly gripping the outer needle hub 3. Consequently, the puncturing operation is easy to carry out, and the burden on the patient's blood vessel is alleviated.
[3] After the flashback is confirmed, the outer needle 2 is further advanced along the inner needle 4, with the inner needle 4 as a guide, by a minute distance in the distal direction.

This operation is carried out by pushing the finger hold part 6 in the distal direction with an index finger (pushing operation). As a result, the outer needle 2, the outer needle hub 3 and the protector 9 are integrally moved in the distal direction relative to the inner needle 4 and the inner needle hub 5.
In addition, at the time of advancing the outer needle 2 in the distal direction, it is preferable to push the finger hold part 6 in the distal direction while lifting up (in the manner of lifting up) the finger hold part 6 in its projection direction (the upward direction in FIG. 4) by the index finger, thereby moving the outer needle 2 in the distal direction. This ensures that the center axis O₁ of the outer needle 2 can be more reliably prevented from being inclined.
Further, the tube 7 is connected to a proximal portion of the outer needle hub 3, and in the assembled state, the center axis O₁ of the outer needle 2 and the center axis O₂ at the distal portion of the tube 7 are substantially parallel to each other. Therefore, the tube 7 does not obstruct puncturing operations by the outer needle 2 and the inner needle 4, and excellent operability is secured.
[4] When the blood vessel has been captured by the outer needle 2 (i.e., when the outer needle 2 has been moved to a target position), the outer needle 2 or the outer needle hub 3 is fixed by one hand, while the inner needle hub 5 is gripped and pulled in the proximal direction by the other hand. Consequently, a series of operations (motions) ranging from a motion of pulling the inner needle 4 out of the outer needle 2 to disengagement of the protector 9 from the outer needle hub 3 are carried out sequentially and continuously. More specifically, first, the inner needle 4 is moved in the proximal direction, and then the inner needle 4 is pulled out from the outer needle 2.
When the inner needle 4 has further been moved in the proximal direction and the needle tip 41 has passed through the slit 81, the seal member 8, which has a self-closing property, closes the slit 81 under its own elastic force. This ensures that leakage of liquid though the slit 81 can be prevented from occurring, whereby an aseptic condition inside the outer needle hub 3 is assured.
When the inner needle 4 is moved further in the proximal direction until the needle tip 41 reaches the proximal side of the shutter member 94, return movement of the needle tip 41 of the inner needle 4 in the distal direction is inhibited, since the needle tip 41 abuts on the shutter member 94 under the action of the shutter member 94 mentioned above. When the inner needle 4 is moved further in the proximal direction and the inner needle 4 is engaged with the inner member 93 of the protector 9, the inner member 93 becomes movable in the proximal direction relative to the protector cover 92. When the inner member 93 is moved in the proximal direction relative to the protector cover 92 until a distal portion of the inner member 93 reaches the proximal side of the projections 923 of the protector cover 92, the projections 923 become movable toward the center axis of the inner needle 4, whereby engagement or catch between the projections 923 and the edge portions confronting the holes 35 is released, as above-mentioned. Thereafter, the inner member 93 and the protector cover 92 are integrally moved in the proximal direction, and the protector 9 is separated (disengaged) from the outer needle hub 3.

[5] Subsequently, the tube 7, which is inserted in the tube-containing section 52 of the inner needle hub 5, is detached through the groove 521.
After the inner needle 4 has been pulled out from the outer needle 2 in this manner, the inner needle 4 and the inner needle hub 5 are rendered useless, and the inner needle 4 and the inner needle hub 5 are therefore discarded.

The needle tip 41 of the inner needle 4 is covered by the protector 9. More specifically, the needle tip 41 is prevented from moving toward the distal side and beyond the shutter member 94 in such a manner as to protrude from the distal end of the protector 9. Therefore, an accident, in which a person in chare of discarding the inner needle 4 or the like might pierce his or her finger with the needle tip 41 by mistake, is prevented from occurring.
[6] Next, the wings 12a and 12b are opened, and are fixed to a patient's skin with a pressure sensitive adhesive tape or the like.
[7] In a case where a vacuum blood collection tube is used as the reduced-pressure container 20, blood B in flashback is passed through the interior of the suction power-controlling mechanism 40 (orifice 403) and then is collected.
[8] Subsequently, the connector 72 of the tube 7 is gripped, and the connector 72 is detached from the suction power-controlling mechanism 40.

Then, a connector (not shown) attached to an end portion of an infusion line for infusion is connected to the connector 72 detached from the suction power-controlling mechanism 40. Consequently, an infusion can be supplied from the infusion line, and the infusion is injected into the patient's blood vessel through the respective inner cavities of the connector 72, the tube body 73a, the outer needle hub 3 and the outer needle 2.

### <Second Embodiment>

FIG. 7 is a partial longitudinal sectional view of a suction power-controlling mechanism in a medical device (second embodiment) of the present invention.

Next, the second embodiment of the medical device according to the present invention will be described below referring to this figure. The following description will be centered on differences from the above-described embodiment, and descriptions of the same items common to the above embodiment will be omitted.

The present embodiment is the same as the first embodiment, except for difference in configuration of the suction power-controlling mechanism.

A suction power-controlling mechanism 40A shown in FIG. 7 has a switching valve 50 that is turnably supported on a body part 401 thereof. The switching valve 50 is cylindrical in shape, and can be turned about an axis thereof. The switching valve 50 is formed therein with a flow path 501 penetrating the inside thereof in a radial direction. In addition, the flow path 501 has a constant inside diameter part 502 having a constant inside diameter, and an enlarged part 503 having an inside diameter larger than the inside diameter of the constant inside diameter part 502. In the suction power-controlling mechanism 40A, the constant inside diameter part 502 is located on the proximal side, and the enlarged part 503 is located on the distal side.

The body part 401 is provided therein with three orifices 403a, 403b and 403c in parallel to each other. Of the orifices 403a, 403b and 403c, the orifice 403a located in the center has the largest inside diameter φd1, the orifice 403b has the second largest inside diameter, and the orifice 403c has the smallest inside diameter φd1. Further, the orifice 403a has the shortest length, and the orifices 403b and 403c have the same length which is larger than the length of the orifice 403a.

In the suction power-controlling mechanism 40A as configured above, by rotating the switching valve 50, the constant inside diameter part 502 of the flow path 501 can be caused to correspond to (communicate with) one of the orifices 403a, 403b and 403c. Incidentally, the enlarged part 503 of the flow path 501 communicates with a lumen part 408 of a mouth part 402, irrespectively of the turning position of the switching valve 50.

By turning the switching valve 50, it is possible to select one of the orifices 403a, 403b and 403c, and thereby to cause the selected orifice and the flow path 501 of the switching valve 50 to communicate with each other. Thus, the degree of control of the suction power can be changed depending on the magnitude of the inside diameter φd1 of the orifice selected. Depending on the specification (gauge size) of the indwelling needle assembly 1 or the size (capacity) of the reduced-pressure container 20 or the like, it may be necessary to regulate the degree of control of the suction power. In such a case, the configuration of the suction power-controlling mechanism 40A, namely, the configuration which is capable of changing the degree of control of the suction power, is effective.

Further, in a case of drawing blood by use of a vacuum blood collection tube as the reduced-pressure container 20, operation to cause blood B to pass through the orifice having a large inside diameter can shorten the working time.

### <Third Embodiment>

FIG. 8 is a plan view showing a third embodiment of the medical device according to the present invention.

Next, the third embodiment of the medical device of the present invention will be described referring to this figure. The following description will be centered on differences from the above-described embodiments, and descriptions of the same items as above will be omitted.

The present embodiment is the same as the above-described first embodiment, except for difference in the configuration of the suction means (tube).

In a medical device 10A as shown in FIG. 8, a third tube 7c branching from an intermediate portion of a tube body 73a of a first tube 7a through a branching connector (branching part) 79 is connected. The third tube 7c has a proximal portion that is preliminarily connected to the connector of the above-mentioned infusion line. In addition, a clamp (not shown) is attached to an intermediate portion of the third tube 7c.

In the thus-configured medical device 10A, infusion can be performed by detaching the clamp attached to the third tube 7c and attaching the clamp to an intermediate portion of the first tube 7a.

In addition, in the medical device 10A, when infusion is performed, it is possible to omit the operation of detaching the connector 72 from the suction power-controlling mechanism 40 and then attaching the connector 72 to the connector of the infusion line, as in the case of the medical device 10 of the first embodiment. Therefore, a speedy infusion operation can be performed. In addition, in the medical device 10A, since the operation of detaching and attaching of the connector 72 can be omitted, and thus exposure of the inside of the first tube 7a to the atmospheric air can be prevented. Therefore, an aseptic condition of the medical device 10A (the inside of the first tube 7a) can be maintained.

Incidentally, the branching connector 79 is in the shape of capital "T" in plan view, and the third tube 7c can be connected to one of the three end portions of the branching connector 79.

While the medical device according to the present invention has been described above while referring to the embodiments shown in the drawings, the invention is not limited to such embodiments. The components of the medical device can be replaced by those of arbitrary configurations capable of exhibiting the equivalent functions to the above-mentioned. Further, arbitrary structures may be added thereto.

In addition, the medical device according to the present invention may be constituted by a combination of two or more configurations (features) arbitrarily chosen from the above-described embodiments.

Also, in the present invention, the shape of the slit in the seal member is not limited to being a straight line segment. For example, other shapes including the shape of a cross, capital Y, capital T, capital H, or the like may also be adopted.

In addition, in the present invention, a cap may be provided which is attached to a proximal portion of the outer needle hub after the inner needle is pulled out from the outer needle. Thus, leakage of liquid via the proximal end of the outer needle hub can be reliably prevented. The cap may be formed integrally with the outer needle hub or may be formed separately from the outer needle hub. Further, a method of fixing the cap to the outer needle hub may be any method such as, for example, a method by use of friction, a method by use of hooking, or the like.

In addition, in the present invention, the protector is not limited to the configuration as shown in the drawings above. The protector may comprise any structure, insofar as it can be detachably connected to the outer needle hub. Particularly, protectors of various configurations can be used, insofar as at least the needle tip of the inner needle is covered therewith when the inner needle is pulled out from the outer needle.

In addition, the reduced-pressure container that can be substituted by a vacuum blood collection tube has been used as the suction source of the suction means. However, the present invention is not limited to this configuration. For example, a syringe having an outer tube and a gasket, a suction pump (a vacuum pump, etc.), a hollow spherical body (balloon) formed of an elastic material, or the like can also be used.

### Industrial Applicability

The medical device according to the present invention includes an indwelling needle assembly which includes an inner needle having a sharp needle tip at the distal end thereof and a hollow outer needle into which the inner needle is inserted, and suction means by which blood is sucked through the indwelling needle assembly in an indwelling state where the indwelling needle assembly is caused to puncture a living body and to indwell therein, wherein the suction means has a suction power-controlling mechanism for controlling the suction power thereof. Therefore, the medical device is simple in structure, the suction power can be maintained for a predetermined period of time, and flashback indicative of capture of a blood vessel can be confirmed speedily. Accordingly, the medical device of the present invention has industrial applicability.

## Claims

1. A medical device comprising:
an indwelling needle assembly which includes an inner needle having a sharp needle tip at a distal end thereof and a hollow outer needle into which the inner needle is inserted; and
suction means by which blood is sucked through the indwelling needle assembly in an indwelling state where the indwelling needle assembly is made to puncture a living body and to indwell therein;
wherein the suction means has a suction power-controlling mechanism for controlling suction power thereof.

2. The medical device according to claim 1,
wherein between an outer peripheral surface of the inner needle and an inner peripheral surface of the outer needle, a gap communicating with the suction means is formed along a longitudinal direction of the indwelling needle assembly, and blood flows into the gap.

3. The medical device according to claim 2,
wherein in the gap, the suction power controlled by the suction power-controlling mechanism is 5 mmHg or more lower than the atmospheric pressure.

4. The medical device according to claim 1,
wherein the suction power acts before the indwelling state is established.

5. The medical device according to claim 1,
wherein the suction power-controlling mechanism has an orifice.

6. The medical device according to claim 5,
wherein the suction power-controlling mechanism has a plurality of the orifices having different inside diameters, and is capable of selecting one of the plurality of the orifices.

7. The medical device according to claim 1,
wherein the suction means is configured to maintain the suction power thereof for a predetermined period of time.

8. The medical device according to claim 1,
wherein the suction means has a container in which a reduced-pressure condition is maintained.

9. The medical device according to claim 1,
wherein the suction means has a tube connected to the indwelling needle assembly, and
the tube has a connector detachably connected to the suction power-controlling mechanism.

10. The medical device according to claim 9,
wherein when the connector is detached from the suction power-controlling mechanism, the detached connector is capable of being connected to an infusion line for infusion.
